# EUROPEAN PATENT APPLICATION

(11) **EP 1 062 913 A1**
(43) Date of publication of application: **27.12.2000**
(21) Application number: 99112248.2
(22) Date of filing: 25.06.1999
(51) Int. Cl.: A61B 6/00

(54) **Digital scanning and photographic imaging X-ray system**

(71) Applicant: DDI Direct Digital Imaging GmbH, 6003 Luzern (CH)
(72) Inventor: Tretiakov, Vjatcheslav, 630117 Novosibirsk (RU); Skok, Andrei, 630058 Novosibirsk (RU); Portmann, Markus, 6034 Inwil (CH); Geisser, Albert, 6373 Ennetbürgen (CH)
(74) Representative: Blum, Rudolf Emil Ernst

(57) **Abstract**

An integrated photographic and digital X-raying system (1) is disclosed that is useful to create fast digital full body images with low dosage and partial body images with high photographic resolution. The system (1) comprises an X-ray source (2), a cassette holder (4) for analog photographic X-ray imaging, a support structure (9) and, according to invention, an X-ray collimator (3), a single- or multiline X-ray scintillation detector (6, 14), scanning means (7, 9, 11, 15) and a personal computer (16) for digital serial readout from the detector (6, 14). The system (1) shall be switchable, preferably automatically, between photographic and digital operation. Preferred configurations of the digital subsystem are disclosed that allow to minimize the X-ray exposure of patients (5).

## Description

### TECHNICAL FIELD

The invention refers to the field of X-ray imaging for medical and other purposes. It is based on the subject-matter as set forth in the preamble of claim 1.

### BACKGROUND ART

The invention refers to a state of the art as known from commercially available medical X-ray systems. Such systems comprise an X-ray source and cassette holder for photographic films for imaging parts of a patient's body with high resolution. Typically a support is provided for holding the X-ray source and the cassette holder in fixed positions for photographic X-ray imaging. A major disadvantage of these conventional X-ray systems is the limitation of the imaging area by the size of the photographic film and the aperture of the X-ray beam. Consequently it is impossible to create quickly and efficiently a complete X-ray image of a patient's body, as is required after accidents or in other cases of emergency. As well, the development time of the X-ray film severely limits the usefulness of conventional X-ray systems in cases where quick medical decisions are urgently needed. Furthermore a dark room and equipment for film development and extended archives for storage and retrieval of X-ray photographies are necessary.

In the U. S. Pat. No. 4,628,356 a digital X-ray scanning system for dental X-ray imaging is disclosed. The X-ray shadow of teeth is detected with a linear sensing array, such as a CCD or photodiode linear array, in optical communication with a fluorescent screen. The detector may be movable or stationary and the X-ray beam is kept in a relatively stationary position. The scanning is performed by either moving the object or by simultaneously moving slit apertures in front of and behind the object. Obviously only small areas may be scanned in such a way and the system is incapable of producing full body images.

In the EP 0 291 299 a digital X-ray scanning system for full body imaging is shown. A scintillator and multi-line photodetector are scanned synchronously with a moving X-ray beam across the stationary patient. A major problem is the image resolution which is considerably lower than in conventional photographic X-raying systems.

### BRIEF SUMMARY OF THE INVENTION

It is the object of the invention to provide an improved X-ray system for fast, large-area as well as high resolution X-ray imaging. This object is achieved according to the invention by the subject-matter as set forth in claim 1.

The invention resides in an X-ray system, steered by a control unit, with an X-ray source and a cassette holder for photographic films, that are kept by a support in fixed positions for conventional x-ray imaging, whereby additional means for digital X-ray scanning and imaging are provided. By combining a photographic analog X-ray imaging system with a novel digital scanning X-ray system a very versatile medical instrument is created. The digital subsystem serves for fast X-raying parts or the totality of a human or animal body or other extended object. Such X-ray scans may be monitored on a TV screen and/or may be image processed, stored, archived and retrieved electronically. The photographic subsystem serves for investigating in greater detail and with higher resolution specific body parts or object details. It is particularly useful for imaging bone structures and performing special examinations, such as mammography. Both subsystems are integrated into one single apparatus and advantageously utilize the same X-ray source.

In one embodiment the photographic and digital X-ray subsystem can selectively be activated by the control unit or by a sensor or a switch in the cassette holder or by means of software.

In another embodiment an X-ray collimator and a pixeled X-ray detector for digital imaging are scanned in coordination with the X-ray source over an object area and means for digital data acquisition from the pixeled X-ray detector are provided.

In yet other embodiments configurations of the digital subsystem are disclosed that allow to minimize the X-ray exposure of patients.

Further embodiments refer to the pixeled X-ray detector comprising a single- or multi-line array of scintillator crystals that are coupled to optical detectors, to an A/D converter and to a personal computer for serial readout.

Other objects, features and advantages of the present invention will become apparent from the dependent claims and the description in connection with the drawings.

### BRIEF DESCRIPTION OF THE DRAWINGS

The description is related to the accompanying drawings, in which, according to invention,
- Fig. 1: shows a first embodiment of the integrated photographic and digital X-ray system,
- Fig. 2: shows a cassette holder with a built-in digital scanning detector,
- Fig. 3: shows a second embodiment of the integrated photographic and digital X-ray system and
- Fig. 4: shows a configuration optimized for low radiation exposure during digital X-ray scanning.

In the drawings identical parts are designated by identical reference numerals.

### DESCRIPTION OF THE PREFERRED EMBODIMENTS

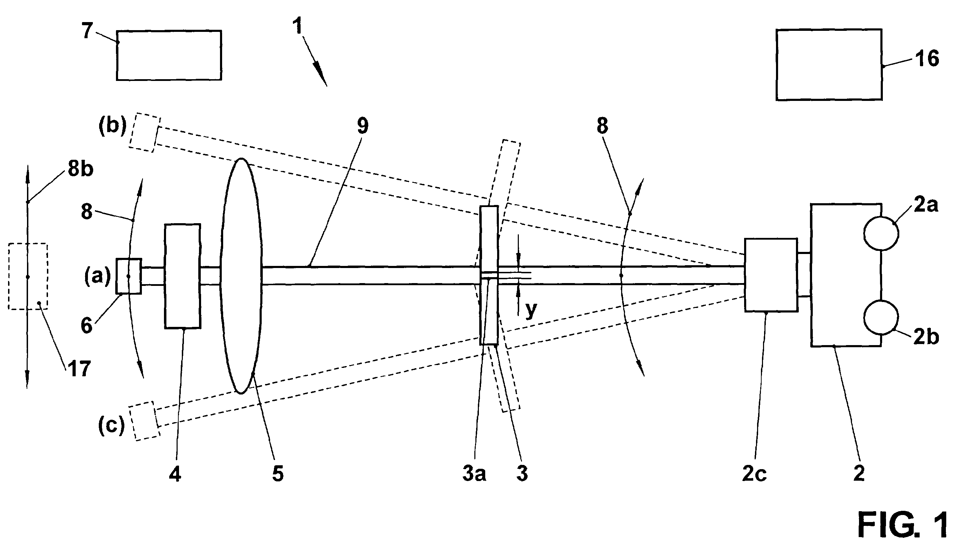

The invention refers to an X-ray system 1 as exemplified in Fig. 1-4. The system 1 comprises an X-ray source 2 with an anode 2a and a cathode 2b (indicated schematically) and a cassette holder 4 for photographic films, a support for holding the X-ray source 2 and the cassette holder 4 in fixed positions for photographic X-ray imaging and a control unit 2c for steering the X-ray system. According to invention additional means 3, 3a, 6, 7, 14, 16, 17 for digital X-ray scanning and imaging are provided. In the following preferred embodiments are described.

The X-ray system 1 shall be switchable between photographic and digital X-ray imaging. In particular means for switching between photographic and digital X-ray imaging and means for steering the digital X-ray scanning are provided. Thus both photographic and digital imaging can be performed using the same X-ray tube 2.

The X-ray system 1 shall comprise an X-ray collimator 3, a pixeled X-ray detector 6, 14, means for coordinately moving the X-ray source 2, the X-ray collimator 3 and the pixeled X-ray detector 6, 14 for scanning an area 5 and means 16 for digital data acquisition from the pixeled X-ray detector 6, 14. In this digital scanning configuration large areas 5 can be sweeped with high precision using a small X-ray beam profile. Thereby the radiation exposure is kept low.

Preferably the X-ray collimator 3 has a slit 3a with adjustable width y for optimally adapting the beam width to the detector pixel size. The X-ray collimator 3 shall be removable or the slit 3a be openable for allowing a broad beam during photographic X-ray imaging. In particular the X-ray collimator 3 or the slit 3a shall be steered by a sensor indicating the presence of a photographic film in the cassette holder 4. By this design, according to invention, a rectangular beam profile is provided that can easily and efficiently be widened or narrowed for photographic or digital imaging.

For the pixeled X-ray detector 6, 14 a single- or multi-line X-ray detector 6, 14 with X-ray sensitive elements (not shown) may be chosen. In particular the X-ray sensitive elements comprise scintillator crystals and optical detectors, that are connected to at least one A/D converter and to a microcomputer 16 for serial readout. By a careful choice of scintillators and detectors a high sensitivity can be achieved. A single- or multi-linear array is clearly superior to full image detectors that require enormous parallel computing power for readout. In contrast the invention takes advantage of a fast serial pixel readout that can be accomplished with commercially available personal computers. The serial readout is repeated line-wise during the scanning process.

In further embodiments the single- or multi-line X-ray detector 6, 14 has means for timing control of the single- or multi-line X-ray detector 6, 14. The single- or multi-line X-ray detector 6, 14 has means for gain and/or offset correction of analog signals from each pixel and/or from the whole single- or multi-line X-ray detector 6, 14. With advantage the single- or multi-line X-ray detector 6, 14 has a digital signal processor for detector control and data acquisition and/or it has a digital memory for data acquisition and data storage.

In Fig. 1, 3 and 4 the support is partially shown. In a preferred embodiment it comprises a transverse bar 9 suspended movably in vertical direction and rotatably, preferably by ±90°. The transverse bar 9 carries the X-ray source 2, the X-ray collimator 3, the cassette holder 4 and the single- or multi-line X-ray detector 6, 14. In particular the X-ray source 2, the X-ray collimator 3, the cassette holder 4 and the single- or multi-line X-ray detector 6, 14 are movable along the transverse bar 9 and are tiltable with respect to the transverse bar 9.

In Fig. 1 the scanning is done by a vertical swivelling movement 8 essentially about the X-ray source 2. The transverse bar 9 in the middle position (a) (straight lines) is oriented horizontally or vertically depending on whether the patient 5 is standing/sitting or lying. The bar 9 may also be oriented under arbitrary angles relative to the patient 5. The dashed lines indicate the extreme positions (b), (c) of the swivelling movement 8. Owing to the bar 9 the detector 6 (with or without cassette holder 4) and the X-ray collimator 3 travel coordinately on a circle segment. This motion might also be achieved using separate mountings for the detector 6 and collimator 3. Alternatively the detector 17 may travel along a straight line segment 8b in coordination with the swivelling movement of the X-ray collimator 3 and X-ray source 2. The movements shall be driven by at least one motor 7.

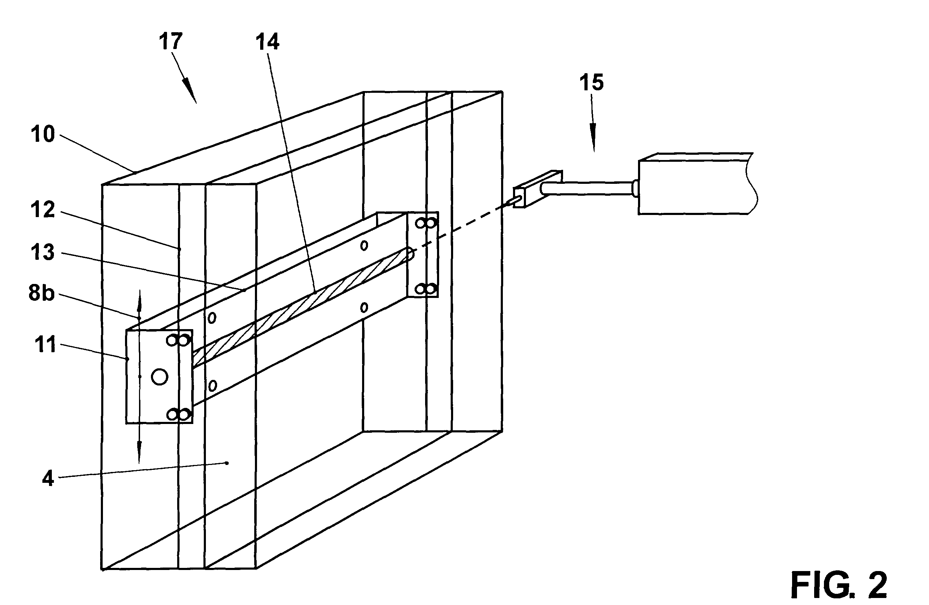

Fig. 2 shows a preferred detector arrangement 17 in greater detail. The single- or multi-line X-ray detector 14 may be placed behind the cassette holder 4, which is shown only in a general simplified manner and is a conventional holder for X-ray films. The detector 14 may as well be placed in front of the X-ray film holder 4, if it can be moved out of the way during the film exposure. The detector 14 may be accomodated together with the cassette holder 4 in a common housing 10 to form an integrated photographic/digital detector 17. As shown the detector 14 is preferably mounted inside the housing 10 on an elongated carriage 11 that is perpendicularly movable along a straight line segment 8b of guiding rails 12. The carriage 11 comprises a rotatable plate 13 that carries the detector 14 and is tilted towards the X-ray source 2 by a motor drive unit 15 in coordination with the up/down-movement 8b (patient standing/sitting) or backward/forward-movement 8b (patient lying) of the carriage 11 inside the housing 10 and/or of the housing 10 itself.

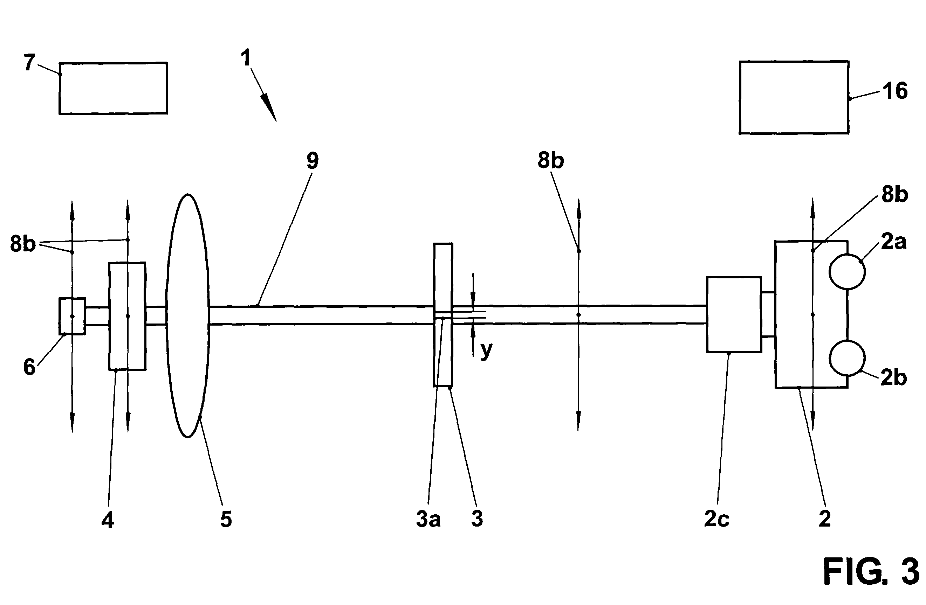

Fig. 3 shows an alternative scanning movement where the transverse bar 9 shifts the X-ray source 2, X-ray collimator 3, cassette holder 4 and digital detector 6 (or integrated photographic/digital detector 17) in straight direction 8b up/down (patient standing/sitting) or backward/forward (patient lying). This is most easily accomplished by moving the suspension arrangement of the transverse bar 9 along a straight supporting part (not shown).

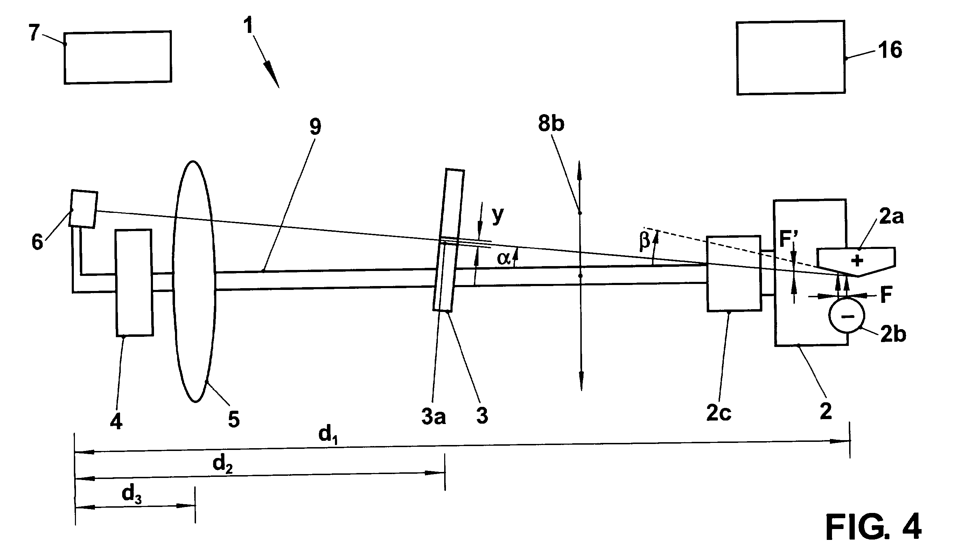

Fig. 4 shows a configuration for minimizing the X-ray dosage during the digital X-ray scan. In this embodiment the single- or multi-line X-ray detector 6 (or 14, not shown) is mounted in a position shifted towards an anode side 2a of the X-ray source 2 by an angle α, where 0°<α<β with β=anode angle. Note that the anode 2a is shown in actual shape and orientation, but not to scale, whereas the cathode 2b is only indicated schematically.

The optimal choice of positioning angle α results from a trade off between emitted intensity and apparent focal spot size F'. The intensity emitted under anode angle β, i. e. tangentially to the anode surface, is considerably filtered and decreased, mainly owing to the surface roughness of the anode material. The intensity strongly increases for decreasing positioning angles α<β and typically reaches 90% of its maximal value at α approximately equal to β/2. On the other hand the apparent focal size equals F'=F*sin(β-α)/sin(β) with F=actual focal spot size. F' is minimal, or ideally zero, for α=β and increases with decreasing positioning angles α<β. Preferably the positioning angle shall be chosen around α=β/2 for receiving a high-intensity X-ray beam with focal spot size reduced by a factor 2. Conventional X-ray tubes 2 have anode target angles β between 12° and 16°. Consequently a useful range of positioning angles is 4°<α<12°, preferred 6°<α<8°, and most preferably α=6° when β=12° is assumed.

According to another embodiment the X-ray dosage and scattering radiation during digital X-ray scanning can be further reduced by an adequate choice of the geometrical beam parameters, such as apparent focal spot size F', collimator slit width y and distances d₁ between apparent focus and detector 6, d₂ between X-ray collimator 3 and detector 6 and d₃ between patient 5 and detector 6. The goals of an optimal parameter choice are: (i) fan beam width at detector site < pixel width (given by scintillator crystal width); (ii) reduce half-shadow zones to diminish radiation passing by the detector 6; thereby the patient's X-ray exposure is further minimized; (iii) provide sufficient absolute intensity; and (iv) small patient-detector distance d₃ to reduce scattering from the X-rayed object. Therefore a slit width y of the order of or smaller than the scintillator crystal width shall be chosen. The half-shadow zones result from the finite apparent focal spot size F' geometrically imaged through the collimator slit 3a. The intensity side lobes extending laterally over more than one pixel size are kept low by decreasing F', y and the image distance d₂. For providing enough absolute intensity the slit width y must be large enough. Finally the patient-detector distance d₃ shall be short. For a pixel size (in particular width of scintillator crystal) of 0.4 mm preferred parameter ranges are: slit width 0.2 mm<y<1.5 mm; overall distance 900 mm<d₁<1300 mm; image distance 500 mm<d₂<700 mm; and patient-detector distance 10 mm<d₃<200 mm.

In conclusion a novel integrated photographic and digital X-raying system is disclosed that is useful to create fast digital full body images with low dosage and partial body images with high photographic resolution.

## Claims

1. X-ray system (1) comprising an X-ray source (2) and a cassette holder (4) for photographic films, a support for holding the X-ray source (2) and the cassette holder (4) in fixed positions for photographic X-ray imaging and a control unit (2c) for steering the X-ray system, characterized in that additional means (3, 3a, 6, 14, 16, 17) for digital X-ray scanning and imaging are provided.

2. The X-ray system (1) according to claim 1, characterized in that
a) the X-ray system (1) is switchable between photographic and digital X-ray imaging and
b) in particular means for switching between photographic and digital X-ray imaging and means for steering the digital X-ray scanning are provided.

3. The X-ray system (1) according to one of the claims 1-2, characterized in that the additional means (3, 3a, 6, 14, 16, 17) comprise an X-ray collimator (3), a pixeled X-ray detector (6, 14), means for coordinately moving the X-ray source (2), the collimator (3) and the pixeled X-ray detector (6, 14) for scanning an area (5) and means (16) for digital data acquisition from the pixeled X-ray detector (6, 14).

4. The X-ray system (1) according to claim 3, characterized in that
a) the X-ray collimator (3) has a slit (3a) with adjustable width y,
b) the X-ray collimator (3) is removable or the slit (3a) is openable for photographic X-ray imaging and
c) in particular the X-ray collimator (3) or the slit (3a) is steered by a sensor indicating the presence of a photographic film in the cassette holder (4) or by a switch in the cassette holder (4).

5. The X-ray system (1) according to one of the claims 3-4, characterized in that
a) the pixeled X-ray detector (6, 14) is a single- or multi-line X-ray detector (6, 14) with X-ray sensitive elements and
b) in particular the X-ray sensitive elements comprise scintillator crystals and optical detectors, that are connected to at least one A/D converter and to a microcomputer (16) for serial readout.

6. The X-ray system (1) according to one of the claims 3-5, characterized in that
a) the single- or multi-line X-ray detector (14) is placed behind the cassette holder (4) and/or
b) the single- or multi-line X-ray detector (14) and the cassette holder (4) are accomodated in a common housing (10) and/or
c) the single- or multi-line X-ray detector (14) is mounted inside a housing (10) on an elongated carriage (11) that is perpendicularly movable and coordinately tiltable towards the X-ray source (2).

7. The X-ray system (1) according to one of the claims 3-6, characterized in that
a) the single- or multi-line X-ray detector (6, 14) is mounted in a position shifted towards an anode side (2a) of the X-ray source (2) by an angle α, where 0°<α<β with β=anode angle and
b) in particular 4°<α<12°, preferably α=6° with β=12°.

8. The X-ray system (1) according to one of the claims 3-7, characterized in that the single- or multi-line X-ray detector (6, 14) has means for gain and/or offset correction of analog signals from each pixel and/or from the whole detector (6, 14).

9. The X-ray system (1) according to one of the claims 3-8, characterized in that
a) the single- or multi-line X-ray detector (6, 14) has a digital signal processor for detector control and data acquisition and/or
b) the single- or multi-line X-ray detector (6, 14) has a digital memory for data acquisition and data storage.

10. The X-ray system (1) according to one of the claims 3-9, characterized in that
a) the support comprises a transverse bar (9) suspended movably in vertical direction and rotatably, preferably by ±90°,
b) the transverse bar (9) carries the X-ray source (2), the X-ray collimator (3), the cassette holder (4) and the single- or multi-line X-ray detector (6, 14) and
c) in particular the X-ray source (2), the X-ray collimator (3), the cassette holder (4) and the single- or multi-line X-ray detector (6, 14) are movable along the transverse bar (9) and are tiltable with respect to the transverse bar (9).
